Europäisches Patentamt

European Patent Office

Office européen des brevets

(19)

(11) Publication number: **0 455 904 A2**

# (12) EUROPEAN PATENT APPLICATION

(21) Application number: 90309670.9

(51) Int. Cl.⁵: **C12M 1/26**

(22) Date of filing: 04.09.90

(30) Priority: 09.05.90 US 521685

(43) Date of publication of application:
**13.11.91 Bulletin 91/46**

(84) Designated Contracting States:
**AT BE CH DE DK ES FR GB GR IT LI LU NL SE**

(71) Applicant: **MICROPROBE CORPORATION**
**1725-220th Street S.E. Suite 104**
**Bothell, WA 98021(US)**

(72) Inventor: **Layton, Tamara J.**
**8010-18th Street, N.W.**
**Seattle, Washington 98117(US)**
Inventor: **Motley, Stanley T.**
**23825-80th Avenue**
**West Edmonds, Washington 98020(US)**
Inventor: **Adams, Trevor H.**
**18440-199th Street N.E.**
**Woodinville, Washington 98072(US)**
Inventor: **Robertson, Rex E.**
**17622-158th Place S.E.**
**Monroe, Washington 98272(US)**

(74) Representative: **Thomson, Paul Anthony et al**
**Potts, Kerr & Co. 15, Hamilton Square**
**Birkenhead Merseyside L41 6BR(GB)**

(54) **Microbiology sampling device.**

(57) A microbiological sampling system and methods for its use. The system comprises an elongated tube member having a tapered inlet end and an outlet end separated by a filter system which traps bacteria. The tapered inlet is used to collect bacteria by application of a negative pressure or vacuum to the outlet end. After collection, the system is removed from the vacuum source and the bacteria is flushed out the inlet end into a sample compartment by filling the outlet end with an aqueous solution and letting the solution flow through the filter. The solution carrying the bacteria exits the inlet end into a sample compartment. The system has particular use in sampling subgingival periodontal pockets.

## BACKGROUND OF THE INVENTION

The present invention relates to a vacuum aspiration system for collecting microbiological flora. The device will find application in a wide variety of medical diagnostic situations where sampling from a lesion, pocket, or canker is required. In particular, this invention provides for a system specifically designed to remove the entire contents of subgingival periodontal pockets in order to allow subsequent analysis of the microbial colonization.

It has long been recognized that there is a clear association between certain bacterial species and the various forms of periodontal disease such as localized juvenile periodontitis (LJP), gingivitis, chronic periodontitis, and acute necrotizing ulcerative gingivitis (ANUG). Bacteria isolated from sites of supposed infection are usually analyzed by classical microbiological techniques to determine if these sites are actually microbiologically active, and if so, with what specie(s) of bacteria. Recent advances in immunoassays and in DNA and RNA oligonucleotide hybridization (Probe) technology now allow the species specific identification of bacteria such as those collected from subgingival periodontal pockets. In contrast to bacterial analysis by classical microbiological technique (Culture), analysis by immunoassay or by Probe technology can be accomplished regardless of the viability of the bacteria.

In the past, microbiological samples from subgingival periodontal pockets have been obtained by various mechanical techniques which vary widely in reproducibility and efficiency. The most commonly used devices are curettes, scalers, and paper (or endodontic) points. They remain as probably the most commonly used sample collection devices due to their ready presence as dental tools. In addition, the variety of shapes and designs of scalers and curettes allows a choice of tools by the hygienist for access to particular periodontal locations. On the other hand, these tools can cause a great deal of variability in sample collection from one practitioner to the other and from one collection episode to another. The design of curettes and scalers is such that the entire contents of a periodontal pocket usually cannot be removed with one scrape. It is common to have the sample fall from the tool during the process of removing the sample from the mouth cavity. It is also common to lose the sample while drawing the curette across adjacent tooth surfaces. Therefore, the quality of sample removal with these tools is often a function of the manual dexterity and care of the professional.

Tools such as curettes or scalers have relatively narrow tip widths - about 0.040" or less, and because periodontal pockets can often contain sample volumes of up to 150 microliters, curettes do not provide a good practical surface to raise the sample from the pocket. Even worse, the tips become worn away during the usable life of the tool. Because of this tool design, the various sample types do not readily adhere to the tool tip. This is because pocket samples can be extremely variable in terms of volume as well as consistency. The samples - even for the same patient - can vary in consistency from rather solid to extremely viscous material due to the presence of blood. It is clear that samples which have a relatively solid consistency are somewhat compatible with the curette design, but the tool is not particularly suited for samples which contain blood or other liquified components. For these reasons, periodontal scalers or curettes are not the preferred sample collection devices for periodontal pockets.

Paper absorbent points are also commonly used to remove samples from periodontal pockets. Paper points are inserted into pockets to allow the contents to rise into and around the paper point fibers by capillary action. These devices are inexpensive and, by their nature, can be inserted into a variety of pocket sizes, depths, and periodontal locations. Yet paper points have probably more serious drawbacks than curettes or scalers. For instance, because the sample collection action is based on capillary attraction, paper points generally remove only the loose bacteria and leave the bacteria which are attached to the tooth surfaces and tissue behind in the pocket. Studies have generally shown that curettes or scalers remove a greater amount of the total microbiological flora than paper points. Because the sensitivity of the bacterial identification method is an important factor in diagnosis and prognosis of the periodontal disease, and because the sensitivity of the method is directly dependent on the disclosed sample collection system efficiency, it follows that paper points are not ideal devices for this purpose.

## SUMMARY OF THE INVENTION

The present invention is directed to a bacterial collection system which solves a longstanding problem regarding a uniform means to assay from and quantify the bacterial contents of a subgingival periodontal pocket.

The system has general application to the sampling of bacteria from a biological matrix such as, tissues, organs or bone. In particular, the device has a tapered distal portion which is contacted onto the matrix. A gentle negative pressure or vacuum is applied to draw bacterial containing fluid or debris into the distal passageway wherein the bacteria are captured by a filter system. The system is then removed from the vacuum line and inverted. The proximal end is filled with a wash solution which flushes the bacteria out and into a collection compartment. The bacteria are then analyzed in any number of standard ways including culturing, immunoassays and nucleic acid hybridization technology.

The system may be used to sample tissue from a bacterial infection on any part of a body. However, in its preferred use, the system will collect bacteria from a subgingival periodontal pocket to assay for periodontal disease.

The device described herein is particularly designed to collect bacteria from subgingival periodontal pockets for subsequent analysis of the pocket contents, such as by nucleic acid probe analysis. The device has been designed to incorporate filter materials housed within a small tip of molded plastic or other material which can be attached directly to a dentist's or dental hygienist's in-house vacuum device which is otherwise used for aspiration of saliva. After a periodontal subgingival pocket has been identified, the pocket contents may be loosened using a standard dental scaler or curette, and then the vacuum sampling device may be used to remove the pocket contents for subsequent analysis.

Other features and advantages of the invention will appear from the following description in which the preferred embodiment has been set forth in detail in conjunction with the accompanying drawing.

BRIEF DESCRIPTION OF THE DRAWING

FIG. 1 is a perspective of the preferred embodiment of the bacterial sampling system of the present invention.

FIG. 2 is a cross-sectional view of the system taken along line 15-15 of FIG. 1.

FIG. 3 is a perspective of the preferred embodiment illustrating its use with a vacuum device.

DETAILED DESCRIPTION OF THE PREFERRED EMBODIMENT

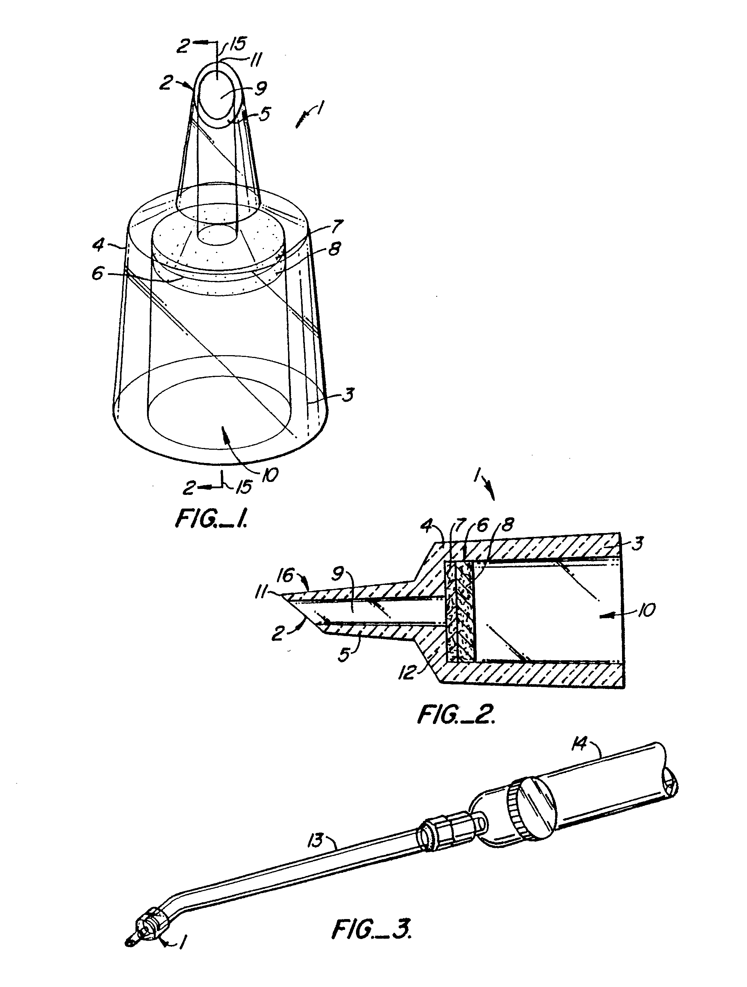

Referring to FIG. 1, there is shown a bacterial sampling device 1 comprising in general an elongated tube member having an inlet end 2 and an outlet 3 end. The elongated tube member has a tapered end to form a nozzle 5. The nozzle 5 has a non-tissue cutting and gently rounded tip 11 preferably cut to form an internal angle 16 of between 35° and 55° and a external diameter of between about 2 to 5 mm. The nozzle 5 forms the exterior of a distal passageway 9 which at its tip 11 forms an elliptical opening having its longest axis between about 1 to about 2 millimeters. For periodontal applications, the distance between the tip 11 and filter 7 should be minimized and is preferably approximately 7 to 12 mm in length.

As can be seen by FIG. 2, the device 1 further comprises an intermediate portion 4 which houses a filter assembly 6. The filter assembly 6 is designed to screen and trap bacteria. Its maximum average pore size is about 150 microns and is preferably about 25 to 200 microns. The filter assembly 6 is preferably comprised of a first filter 7 and second filter 8. The first filter 7 is a prefilter designed to permit at least some, and preferably substantially all, bacteria to pass through it while entrapping larger material such as dust, blood cells, patient tissues and the like. The first filter 7 is generally biologically inert and preferably uncharged at neutral pH. It may be made of a number of materials including but not limited to nylon, glass, ceramic, cellulose, polypropylene, polystyrene, polyethylene, other plastics and natural fiber materials. It is preferably made from 8S Rayon (Schleicher & Schull of Keene, New Hampshire). The second filter 8 traps most bacterial cells and precludes their passage from the distal passageway 9 to the proximal passageway 10. The second filter 8 is generally biologically inert and uncharged at neutral pH. The second filter 8 is preferably adjacent to the first filter 7. It may be made of a number of materials including but not limited to nylon, glass, ceramic, cellulose and other plastic and natural fiber materials. It is preferably made from Porex HDPE filter material (Porex Technologies of Fairburn, Georgia).

The filter assembly 6 should be firmly secured to the internal walls of the intermediate portion. The means for securing the filter assembly are well known in the art. They include an internally threaded internal wall of the intermediate portion 4 with an externally threaded collar, an o-ring seated into a concentric well cut into the internal wall of the intermediate portion, or the filter material can be fused to the internal wall by an epoxy glue or by welding. The preferred means is to ultrasonically weld the filter assembly 6 to the wall. The intermediate portion 4 is preferably stepped from the nozzle 5 whereby the internal diameter of the proximal passageway 10 is increased. The step 12 may be used to position the filter assembly 7 and preclude its forward motion during the flushing step as described below.

The intermediate portion 4 leads to the outlet end 3 forming the proximal passageway 10. The outlet end 3 is adapted and designed to reversibly couple with a vacuum line 13 as can be seen in FIG. 3. The coupling may include any number of standard means such as a friction fitting of soft and pliable plastic pressed upon an inflexible tapered member, a male and female thread assembly, a luer-type fitting, a press and snap fitting or a twist and lock fitting. The frictional fitting is preferred.

As can be seen in FIG. 3, a vacuum line (not shown) is attached to a hand held suction hose fitting 14. The hand held suction hose fitting provides the negative pressure needed to draw the bacteria up into the distal passageway 3 and into the filter assembly 6. The amount of pressure is preferably about 8-14 PSI and typically about 12 PSI.

The outlet end 3 is adapted to receive the aqueous solution used to flush the captured bacteria out the inlet end 2. The outlet end 3 forms a proximal passageway 10 which is of a diameter which permits the washing solution to flow freely through the filter. The outlet end 3 is preferably circular having a cylindrical passageway 10 with an internal diameter of about 4 to 10 mm. Some samples may require the application of a slight pressure to allow the washing solution to flow through the filters.

The collection system 1 is particularly designed to find application in the detection of bacteria from a subgingival periodontal pocket. The size and angle of the nozzle preferably permits the dentist to lift the sample without slicing or damaging the surrounding tissue. The distal passageway is of a size that the entire pocket diameter may be sampled without concern that adjacent pockets are being sampled. By attaching the collection system 1 to a vacuum tube 13 which is preferably rotably attached to the suction hose fitting 14, one can readily adjust the angle of the system 1 to sample bacteria from pocket sites which are lingual or buccal.

The various elements of the system are composed of biologically inert materials. These include metals such as stainless steel or aluminum, ceramics, silicone polymers or plastics. The preferred plastic is a non-wettable acrylic plastic available from Rohm and Haas under the name DR-100.

In use, the dentist gently contacts the nozzle to the periodontal pocket and allows the negative pressure from the standard vacuum line associated with dental aspirators to collect the entire contents of the pocket including the bacteria. A pre-scraping with a curette or dental probe may be useful to loosen the debris before removal. The collection system 1 is then removed from the vacuum line 13 and placed nozzle end down into a sample compartment. A washing solution is introduced into the proximal passageway 10 to flush the bacteria from the filter, out of the distal passageway 9 and into the sample compartment. The sample compartment can be any tube or vial able to hold the washing solution and bacteria.

The washing solution may be any number of aqueous solutions. They may be physiologically compatible if the bacteria are to be sustained in culture. They may be a lysing solution which cause the bacterial cells to open and release their contents. The lysing solution is preferable for those assays which rely upon internal material such as nucleic acid hybridization assays. Such assays are generally described in Nucleic Acid Hybridization A Practical Approach Ed. by B.D. Hames and S.J. Higgins, IRL Press, 1985. The preferred lysing solution will contain a detergent such as sodium dodecylsulfate or sodium laurylsulfate or N-lauroyl-sarconsine. The preferred lysing solution is 1 milligram/milliliter Proteinase K, 50 millimolar TRIS buffer, 10 millimolar ethylenediaminetetraacetic acid (EDTA), 10 millimolar NaCl, 1% (w/v) sarcosine, and 0.5% (w/v) sodium dodecylsulfate, at pH 8.0.

While several embodiments of the invention have been described, it will be understood that it is capable of still further modifications, and this application is intended to cover any variations, uses or adaptations of the invention, following in general the principles of the invention and including such departures from the present disclosure as to come within knowledge or customary practice in the art to which the invention pertains, and as may be applied to the essential features hereinbefore set forth and falling within the scope of the invention or the limits of the appended claims.

## Claims

1. A bacterial collection system comprising:

an elongated tube member having a tapered inlet end, a intermediate portion containing a filter assembly, and an outlet end;

the inlet end being generally cylindrical and tapered to form a nozzle portion having an external diameter of between about 3 and 5 mm;

the nozzle portion being angled at the distal end from about 35° to about 55° to form a blunt non-tissue cutting tip having an elliptical opening of between about 1 and 2 mm along its longest axis; and

the intermediate portion being disposed between the inlet end and the outlet end and having a passageway for communicating between the inlet end and the outlet end with the filter assembly being disposed along the passageway to halt the travel of bacteria from the inlet end to the outlet end.

2. The system according to claim 1 wherein the filter assembly is comprised of a first filter and a second filter.

3. The system according to claim 2 wherein the first and second filters are adjacent to each other.

4. The system according to claim 2 wherein the first filter is sized to permit at least some bacteria to pass through it.

5. The system according to claim 1 wherein the filter assembly has a filter with an average pore size of 150 microns.

6. The system according to claim 1 wherein the intermediate portion is stepped.

7. The system according to claim 1 wherein the tube member is plastic.

8. The system according to claim 1 which further comprises a vacuum device.

9. The system according to claim 8 wherein the outlet end provides a coupling means to the vacuum device through frictional engagement.

10. The system according to claim 8 wherein the outlet end provides a coupling means to the vacuum device through interlocking means.

11. A method for collecting bacteria from the surface of human tissue comprising:

contacting the tissue with a bacterial collection system having:

an elongated tube member having an tapered inlet end, a intermediate portion containing a filter assembly, and an outlet end;

the inlet end being generally cylindrical and tapered to form a nozzle portion;

the nozzle portion being angled to form a tip; and

the intermediate portion being disposed between the nozzle portion and the outlet end and having a passageway for communicating between the inlet end and the outlet end with the filter assembly being disposed along the passageway to halt the travel of bacteria from the inlet end to the outlet end;

applying a negative pressure to the outlet end of the system; and

flushing the bacteria from the filter assembly, out the inlet end and into a sample compartment using an aqueous wash solution.

12. The method according to claim 10 wherein the filter assembly is comprised of a first filter and a second filter.

13. The method according to claim 12 wherein the first and second filters are adjacent to each other.

14. The method according to claim 11 wherein the negative pressure is about 12 pounds per square inch.

15. The method according to claim 12 wherein the aqueous wash is a bacterial lysing solution.

16. The method according to claim 15 wherein the wash solution comprises detergent.

17. The method according to claim 11 wherein the nozzle portion is angled from about 35° to about 55° to form a blunt non-tissue cutting tip having an elliptical inlet opening of between about one to about two millimeters along its longest axis.

18. The method according to claim 11 wherein the tissue is a subgingival periodontal pocket.

FIG._1.

FIG._2.

FIG._3.